(19) Europäisches Patentamt
European Patent Office
Office européen des brevets

(11) Veröffentlichungsnummer: **0 224 163 B1**

(12) **EUROPÄISCHE PATENTSCHRIFT**

(45) Veröffentlichungstag der Patentschrift: **16.10.91**

(51) Int. Cl.5: **C07C 211/27**, C07D 307/14, A01N 33/04, C07C 211/29, C07C 211/17, C07C 211/48, C07C 211/52

(21) Anmeldenummer: **86115916.8**

(22) Anmeldetag: **17.11.86**

Die Akte enthält technische Angaben, die nach dem Eingang der Anmeldung eingereicht wurden und die nicht in dieser Patentschrift enthalten sind.

(54) **Propylamine, Verfahren zu ihrer Herstellung und ihre Verwendung als Fungizide.**

(30) Priorität: **21.11.85 DE 3541181**

(43) Veröffentlichungstag der Anmeldung:
**03.06.87 Patentblatt 87/23**

(45) Bekanntmachung des Hinweises auf die Patenterteilung:
**16.10.91 Patentblatt 91/42**

(84) Benannte Vertragsstaaten:
**AT BE CH DE FR GB IT LI NL SE**

(56) Entgegenhaltungen:
**EP-A- 0 006 992**
**DE-A- 3 309 720**
**DE-A- 3 415 139**

(73) Patentinhaber: **BASF Aktiengesellschaft**
**Carl-Bosch-Strasse 38**
**W-6700 Ludwigshafen(DE)**

(72) Erfinder: **Rentzea, Costin, Dr.**
**Richard-Kuhn-Strasse 1-3**
**W-6900 Heidelberg(DE)**
Erfinder: **Himmele, Walter, Dr.**
**Eichenweg 14**
**W-6909 Walldorf(DE)**
Erfinder: **Buschmann, Ernst, Dr.**
**Georg-Ludwig-Krebs-Strasse 10**
**W-6700 Ludwigshafen(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

**EP 0 224 163 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Propylamine, Verfahren zu deren Herstellung, ihre Verwendung als Fungizide, fungizide Mittel, die die neuen Wirkstoffe enthalten, Verfahren zur Herstellung solcher fungiziden Mittel sowie Verfahren zur Bekämpfung von Schadpilzen mit diesen Fungiziden.

Es ist bekannt, N-Tridecyl-2,6-dimethylmorpholin als Fungizid zu verwenden (DE 1 164 152).

Es wurde nun gefunden, daß Verbindungen der Formel I

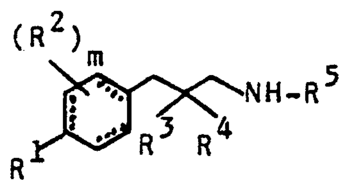

I,

worin

$R^1$ und $R^2$ gleich oder verschieden sind und Wasserstoff, einen Alkylrest mit 1 bis 8 Kohlenstoffatomen, einen Cycloalkylrest mit 3 bis 9 Kohlenstoffatomen, einen Halogenalkylrest mit 1 bis 3 Halogenatomen und 1 bis 4 Kohlenstoffatomen, einen Alkoxyrest mit 1 - 3 Kohlenstoffatomen, Chlor, Brom oder Fluor bedeuten, und

m die ganzen Zahlen 0, 1 oder 2 bedeutet, wobei, falls die gestrichelten Bindungen hydriert sind, $R^1$ einen $C_1$ bis $C_8$-Alkyl- oder $C_3$ bis $C_9$-Cycloalkylrest und $R^2$ Wasserstoff bedeuten, und

$R^3$ und $R^4$ jeweils unabhängig voneinander ein Wasserstoffatom oder einen $C_1$ bis $C_5$-Alkylrest bedeuten, und

$R^5$ einen Alkylrest mit 1 bis 10 Kohlenstoffatomen, einen Cycloalkylrest mit 3 bis 12 Kohlenstoffatomen, einen Cycloalkenylrest mit 5 - 12 Kohlenstoffatomen, einen Cycloalkylalkylrest mit 4 bis 13 Kohlenstoffatomen, einen Alkenylrest mit 3 - 6 Kohlenstoffatomen, oder einen gegebenenfalls durch Halogen, $C_1$ bis $C_4$-Alkyl, Halogen-$C_1$-$C_4$-alkyl, $C_1$-$C_4$-Alkoxy oder Cyan substituiertes Phenyl- oder Phenyl-$C_1$-$C_6$-alkylrest bedeuten, und die gestrichelten Bindungen hydriert sein können, sowie deren Salze, außer den Verbindungen, in denen die gestrichelten Bindungen nicht hydriert sind, $R^1$ einen Isopropylrest, m die Zahl 0 und $R^5$ einen Cycloalkyl-, Bicycloheptylmethylrest oder einen Alkylrest bedeutet und außer den Verbindungen, in denen die gestrichelten Bindungen hydriert sind, m die Zahl 0 und $R^5$ einen Propylrest bedeutet, ausgezeichnet wirksam gegen Schadpilze sind und gute Pflanzenverträglichkeit zeigen.

Die neuen Amine der Formel I enthalten ggf. chirale Zentren. Sie werden im allgemeinen als Racemate und gegebenenfalls als Diastereomerengemische erhalten. Einheitliche Diastereomere lassen sich bei einigen der neuen Verbindungen beispielsweise durch Säulenchromatographie oder aufgrund von Löslichkeitsunterschieden in reiner Form isolieren. Aus solchen gereinigten Diastereomeren kann man mit bekannten Methoden einheitliche Racemate und Enantiomere erhalten. Alle diese Verbindungen und Gemische werden von der vorliegenden Erfindung umfaßt. Für die Anwendung der neuen Amine als Fungizide sind sowohl die einheitlichen Diastereomeren bzw. Enantiomeren wie auch deren bei der Synthese anfallenden Gemische geeignet. Bevorzugt werden die letzteren verwendet.

$R^1$ und $R^2$ bedeuten beispielsweise Wasserstoff, Fluor, Chlor, Brom, Trifluormethyl, Methoxy, Ethoxy, Propoxy, Methyl, Ethyl, geradkettiges oder verzweigtes Propyl, Butyl, Pentyl, Hexyl, Heptyl, Octyl, Cyclopentyl, Cyclohexyl, Methyl-, Dimethyl- und Trimethylcyclohexyl, Norbornyl.

$R^3$ und $R^4$ bedeuten beispielsweise Wasserstoff, Methyl, Ethyl, geradkettiges oder verzweigtes Propyl, Butyl und Pentyl.

$R^5$ bedeutet beispielsweise $C_1$ - $C_{10}$-Alkyl, Methyl, Ethyl, geradkettiges oder verzweigtes Propyl, Butyl, Pentyl, Hexyl, Heptyl, Octyl, Nonyl, Decyl, $C_2$ - $C_6$-Alkenyl, Allyl, Methallyl, Pentenyl, Hexenyl, $C_3$ -$C_{12}$-Cycloalkyl, Cyclopropyl, Cyclopropylmethyl, Cyclopentyl, Methylcyclopentyl, Cyclopentylmethyl, Cyclohexyl, Cyclohexylmethyl, Cyclohexylethyl, Cyclohexylpropyl, Di-, Tri- und Tetramethylcyclohexyl, Ethylcyclohexyl, Propyl- und Isopropylcyclohexyl, Butyl, Isobutyl, sek.-Butyl- und tert.-Butylcyclohexyl, tert.-Amylcyclohexyl, Cyclohexylcyclohexyl, Menthyl, Phenyl-cyclohexyl, Cycloheptyl, Cycloheptylmethyl, Methylcycloheptyl, Propylcycloheptyl, Cyclooctyl, Cyclododecyl, Cyclododecylmethyl, $C_5$ - $C_{12}$-Cyclcalkenyl, Cyclopentenyl, α-Campholenyl, Cyclohexenylmethyl, tert.-Butylcyclohexenyl, tert.-Butylcyclohexylpropyl-, tert.-Butylcyclohexenyl-, tert.-Butylcyclchexenylpropyl, Cycloheptenyl, Cyclooctenyl, Cyclododecadienyl, Cyclododecadienylmethyl, Decalyl, Norbornyl, Tricyclodecanyl, 1,5-Dimethylbicyclo[2,3,1]octan-8-yl, Isobornyl, Adamantyl, Norbornylmethyl, Camphenyl, Homocamphenyl, Pinanyl, Norborneyl, Nopolyl, Phenyl, Chlorphenyl, Dichlorphenyl, Alkoxyphenyl, Alkylphenyl, Phenyl-$C_1$ - $C_6$-alkyl, Benzyl, Halogenphenyl-$C_1$ - $C_4$-alkyl, Chlorbenzyl, Fluorbenzyl, Methylbenzyl, Ethylbenzyl, Propylbenzyl, Butylbenzyl, Methoxybenzyl, Trifluormethylbenzyl, Phenylethyl, 1-Phenyl-2-$C_1$-$C_2$-alkyl-propyl-3, 1-(4-tert.-Butylphenyl)-2-methyl-propyl-3, Chlorphenylpropyl, Dichlorphenylpropyl, Fluorphenylpropyl, Phenylpropyl, Phenylbutyl, Phenylpentyl und Phenylhexyl, 2-Methylfuran.

Die Amine der Formel I lassen sich herstellen, indem man

a) einen Aldehyd der Formel II mit einem Amin der Formel III

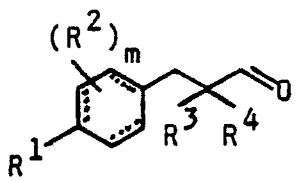

II + $H_2N-R^5$ III,

in welcher $R^1$, $R^2$, $R^3$, $R^4$, $R^5$, m und die gestrichelten Bindungen die oben genannten Bedeutungen haben, oder

b) ein Amin der Formel IV mit einem Aldehyd oder Keton der Formel V

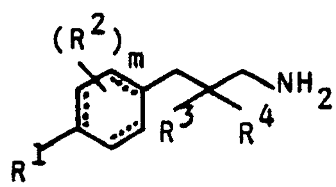

IV + O=C[R'5] V,

in welcher $R^1$, $R^2$, $R^3$, $R^4$, m und die gestrichelten Bindungen die oben genannten Bedeutungen haben und O=C[R'5] der dem Rest $R^5$ entsprechende Aldehyd oder das entsprechende Keton bedeutet außer für $R^5$ = $C_3$-$C_6$-Alkenyl, $C_5$-$C_{12}$-Cycloalkenyl, in Gegenwart von Ameisensäure oder Natriumborhydrid oder Natriumcyanborhydrid oder in Gegenwart von Wasserstoff und einem Katalysator wie Ni, Pd oder Pt umsetzt, oder

c) eine Verbindung der Formel VI mit einem Amin der Formel III

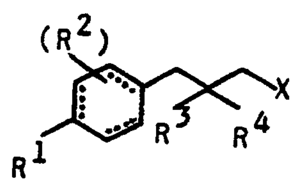

VI + III,

in welcher $R^1$, $R^2$, $R^3$, $R^4$, m, III und die gestrichelten Bindungen die oben genannten Bedeutungen haben und X für eine nucleophil verdrängbare Abgangsgruppe steht, oder

d) ein Alkylierungsmittel der Formel VII mit einem Amin der Formel IV

IV + X-$R^5$ VII,

in welcher IV, X und $R^5$ die oben genannten Bedeutungen haben, umsetzt und die erhaltenen Verbindungen gegebenenfalls in ihre Salze überführt.

Gemäß Verfahrensvariante a) und b) werden Amine der Formel III oder IV mit Aldehyden der Formel II oder V in Gegenwart von Ameisensäure umgesetzt. Diese reduktive Alkylierung wird vorzugsweise in Abwesenheit eines Lösungsmittels durchgeführt. Zur Lösung des Amins in Ameisensäure wird bei Temperaturen von 0 °C bis 110 °C, vorzugsweise 50 - 100 °C der Aldehyd zugetropft.

Dagegen werden die Umsetzungen der Aldehyde II oder V mit Aminen der Formel III oder IV in Gegenwart von Natriumborhydrid oder Natriumcyanborhydrid in einem Lösungs- oder Verdünnungsmittel durchgeführt. Dazu eignen sich vorzugsweise Alkohole wie Methanol, Ethanol, Propanol und Isopropanol,

die bis zu 25 % (Vol.) Wasser enthalten können.

Gemäß Verfahrensvariante a) und b), können Amine der Formel III oder IV mit Aldehyden der Formel II oder V auch in Gegenwart von Wasserstoff und einem Katalysator alkyliert werden.

Als Katalysatoren eignen sich Edelmetalle wie beispielsweise Palladium, Platin - gegebenenfalls auf einem Träger niedergeschlagen - sowie Rhodium und Raney-Nickel. Bevorzugt ist Palladium auf Kohle. Geeignete Lösungsmittel sind Alkohole wie Methanol oder Ethanol, Kohlenwasserstoffe wie Hexan, Heptan, Octan, Cyclohexan, Toluol oder Xylol. Wird eine Perhydrierung angestrebt, so wird als Katalysator Platin in Eisessig unter Zusatz von Perchlorsäure verwendet. Unter diesen Bedingungen wird der aromatische Rest durchhydriert.

Für alle drei Verfahrensvarianten a), b) und c) kommen als Lösungs- oder Verdünnungsmittel beispielsweise Halogenkohlenwasserstoffe, insbesondere Chlorkohlenwasserstoffe, z. B. Tetrachlorethylen-1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1-oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Tri chlorbenzol; Ether, z.B. Ethylpropylether, Methyl-tert.-butylether, n-Butylethylether, Di-n-butylether, Di-isobutylether, Di-isobutylether, Diisoamylether, Diisopropylether, Anisol, Phenetol, Cyclohexylmethylether, Ethylenglykoldimethylether, Tetrahydrofuran, Dioxan, Thioanisol, β,β,β′,-Dichlordiethylether; nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyrnitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Als Lösungsmittel können auch die Verbindungen der Formel III, IV und V im Überschuß verwendet werden. Zweckmäßig verwendet man das Lösungsmittel in einer Menge von 100 bis 2000 Gew.-%, vorzugsweise von 200 bis 700 Gew.-%, bezogen auf Ausgangsstoff II.

Als anorganische oder organische Basen (Säureakzeptoren) für die Umsetzung zu Verbindungen der Formel I können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. als basische Verbindungen in Frage:

Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Tripropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminipyridin, N,N-Dipropyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, β-Picolin, gamma-Picolin, Isochinolin, Pyrimidin, Acridin, N.N.N′,N′-Tetramethylethylendiamin, N,N,N′,N′-Tetraethylethylendiamin, Chinoxalin, Chinazolin, N-Propyldiisopropylamin, N,N-Dimethylcyclohexylamin, 2,6-Lutidin, 2,4-Lutidin, Trifurylamin, Triethylendiamin.

Zweckmäßig verwendet man den Säureakzeptor in einem Überschuß oder Unterschuß von bis zu 20 %, bezogen auf den Ausgangsstoff IV oder VI.

Als Reaktionsbeschleuniger kommen vorzugsweise Metallhalogenide wie Natriumiodid oder Kaliumiodid in Frage.

Als nucleophil verdrängbare Abgangsgruppe X wird beispielsweise Halogen genannt.

Zur Salzbildung mit Verbindungen der Formel I sind alle organischen und anorganischen Säuren geeignet, soweit sie pflanzenphysiologisch verträgliche Salze bilden. So sind z.B. Chloride, Bromide, Jodide, Sulfate, Phosphate, Acetate, Oxalate, Fumarate, Malonate, Alkylsulfonate und Acrylsulfonate, Dodecylbenzylsulfonate zu nennen.

Die Salze werden erhalten, indem man die ensprechende Säure mit freiem Amin der Formel I; gegebenenfalls in einem inerten Lösungsmittel zusammengibt, das Lösungsmittel abtrennt und den Rückstau gegebenenfalls umkristallisiert.

Die Ausgangsstoffe der Formel III, V und VIII sind allgemein bekannt und/oder lassen sich nach an sich bekannten Methoden darstellen. Aldehyde der Formel II und Halogenide der Formel VI (X = Chlor, Brom) sind aus der EP 9977 bekannt.

Die folgenden Beispiele erläutern die Herstellung der Verbindungen der Formel I:

Beispiel 1

a) Einer Lösung von 71 g n-Butylamin in 800 ml Ethanol werden 204 g 2-(4'-tert.-Butylphenyl)-2-methylpropionaldehyd bei 25 °C zugetropft. Nach 36stündigem Rühren bei 25 °C wird das Gemisch portionsweise mit 87 g Natriumborhydrid versetzt und weitere 4 Stunden bei 78 °C gerührt. Das Gemisch wird abgekühlt, mit 1,5 l Wasser verdünnt und dreimal mit je 300 ml Methylenchlorid extrahiert. Die vereinigten Extrakte werden mit 300 ml Wasser gewaschen, getrocknet und destilliert. Man erhält 170 g N-Butyl-N-3-(1-(4'-tert.-butylphenyl)-2 -methyl)-Propylamin vom Siedepunkt 125 °C/0,4 mbar (Verbindung Nr. 1).

b) Eine Lösung von 22,4 g 3-(4'-tert.-Butylphenyl)-2-methylpropylchlorid in 36,5 g n-Butylamin wird mit 2 g Kaliumiodid versetzt und 48 Stunden am Rückfluß gerührt. Nach Abkühlen auf +10 °C wird das Reaktionsgemisch mit 100 ml 20prozentiger (Gew.-%), wäßriger Natriumhydroxylösung versetzt, 10 Minuten bei 25 °C gerührt, die organische Phase getrennt und im Vakuum fraktioniert destilliert. Man erhält 25 g N-Butyl-N-3-(1-(4'-tert.-butylphenyl)-2-methyl)-propylamin vom Siedepunkt 110 °C/0,15 mbar (Verbindung Nr. 1).

Entsprechend können durch Wahl der Ausgangsstoffe und entsprechende Anpassung der Verfahrensbedingungen die nachstehend aufgelisteten Verbindungen erhalten werden:

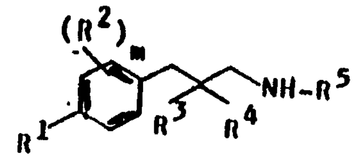

**Tabelle 1**

| Verbindung Nr. | $R^1$ | $R^2$ | $R^3$ | $R^4$ | $R^5$ | Brechungsindex oder Sdp. [°C/mbar] |
|---|---|---|---|---|---|---|
| 2 | tert.-$C_4H_9$ | H | H | $-CH_3$ | $-CH_3$ | 145/10 |
| 3 | tert.-$C_4H_9$ | H | H | $-CH_3$ | $-C_2H_5$ | 118 - 119/3,0 |
| 4 | tert.-$C_4H_9$ | H | H | $-CH_3$ | i-$C_3H_7$ | 130 - 140/5,0 |
| 5 | F | H | H | $-CH_3$ | n-$C_3H_7$ | 80 - 82/0,1 |
| 6 | $-CH_3$ | H | H | $-CH_3$ | n-$C_3H_7$ | 90 - 92/0,1 |
| 7 | tert.-$C_4H_9$ | H | H | $-CH_3$ | n-$C_3H_7$ | 120 - 12/0,2 |
| 8 | Cl | 2-Cl | H | n-$C_4H_9$ | n-$C_3H_7$ | 144/0,1 |
| 9 | $CH_3$-O- | H | H | $-CH_3$ | n-$C_3H_7$ | 128 - 135/2 |
| 10 | $CH_3$-O- | H | H | $-CH_3$ | n-$C_4H_9$ | 130 - 134/0,4 |
| 11 | H | H | H | n-$C_3H_7$ | n-$C_4H_9$ | 110 - 112/0,3 |
| 12 | H | H | H | n-$C_4H_9$ | n-$C_4H_9$ | 124/0,5 |
| 13 | Cl | H | H | $-CH_3$ | n-$C_4H_9$ | 109 - 112/0,4 |
| 14 | H | 2-F | H | $-CH_3$ | n-$C_4H_9$ | 100 - 105/0,4 |
| 15 | Cl | 2-Cl | H | $-CH_3$ | n-$C_4H_9$ | 136 - 140/0,4 |
| 16 | Cl | 2-Cl | H | $-CH_3$ | iso-$C_4H_9$ | 136 - 138/0,2 |
| 17 | Cl | 2-Cl | $-CH_3$ | $-CH_3$ | n-$C_4H_9$ | 122 - 125/0,1 |
| 18 | Cl | H | $-C_2H_5$ | $C_2H_5$ | n-$C_4H_9$ | 137 - 140/0,3 |
| 19 | tert.-$C_4H_9$ | H | H | $-CH_3$ | tert.-$C_4H_9$ | 91 - 100/5 |

Forts. Tab. 1

| Verbindung Nr. | $R^1$ | $R^2$ | $R^3$ | $R^4$ | $R^5$ | Brechungsindex oder Sdp. [°C/mbar] |
|---|---|---|---|---|---|---|
| 20 | $C_2H_5(CH_3)_2C-$ | H | H | $-CH_3$ | $n-C_4H_9$ | 148 - 154/2 |
| 21 | $n-C_3H_7(CH_3)_2C-$ | H | H | $-CH_3$ | $n-C_4H_9$ | 140 - 148/0,4 |
| 22 | 2-Norbornyl | H | H | $-CH_3$ | $n-C_4H_9$ | 164 - 172/0,3 |
| 23 | tert.-$C_4H_9$ | H | H | $-CH_3$ | $n-C_5H_{11}$ | $n_D^{22}$: 1,4886 |
| 24 | tert.-$C_4H_9$ | H | H | $-CH_3$ | $-CH_2CH_2CH(CH_3)_2$ | $n_D^{22}$: 1,4904 |
| 25 | tert.-$C_4H_9$ | H | H | $-CH_3$ | $-CH(CH_3)C_4H_9-n$ | $n_D^{22}$: 1,4878 |
| 26 | tert.-$C_4H_9$ | H | H | $-CH_3$ | $n-C_6H_{13}$ | $n_D^{22}$: 1,4808 |
| 27 | tert.-$C_4H_9$ | H | H | $-CH_3$ | $-CH_2CH(C_2H_5)_2$ | $n_D^{22}$: 1,49903 |
| 28 | tert.-$C_4H_9$ | H | H | $-CH_3$ | $-C(CH_3)_2CH(CH_3)_2$ | $n_D^{23}$: 1,4928 |
| 29 | tert.-$C_4H_9$ | H | H | $-CH_3$ | $-CH(CH_3)CH(CH_3)C_2H_5$ | $n_D^{25}$: 1,4909 |
| 30 | tert.-$C_4H_9$ | H | H | $-CH_3$ | $-CH_2CH(CH_3)C_3H_7-n$ | $n_D^{22}$: 1,4882 |
| 31 | H | H | H | $n-C_3H_7$ | $n-C_6H_{13}$ | 130 - 134/0,5 |
| 32 | H | H | H | $n-C_4H_9$ | $n-C_6H_{13}$ | 145 - 152/0,4 |
| 33 | H | 2-F | H | $-CH_3$ | $-C_6H_{13}$ | 125 - 128/0,4 |
| 34 | Cl | H | H | $-CH_3$ | $n-C_6H_{13}$ | 140 - 143/0,4 |
| 35 | Cl | 2-Cl | H | $-CH_3$ | $n-C_6H_{13}$ | 152 - 154/0,3 |
| 36 | $C_2H_5(CH_3)_2C-$ | H | H | $-CH_3$ | $n-C_6H_{13}$ | 165 - 167/0,5 |
| 37 | $n-C_3H_7(CH_3)_2C-$ | H | H | $-CH_3$ | $n-C_6H_{13}$ | 160 - 162/0,4 |
| 38 | $n-C_4H_9(CH_3)_2C-$ | H | H | $-CH_3$ | $n-C_6H_{13}$ | 172 - 180/0,5 |

Forts. Tab. 1

| Verbindung Nr. | $R^1$ | $R^2$ | $R^3$ | $R^4$ | $R^5$ | Brechungsindex oder Sdp. [°C/mbar] |
|---|---|---|---|---|---|---|
| 39 | $(CH_3)_2CHC(CH_3)_2$- | H | H | $-CH_3$ | n-$C_6H_{13}$ | 159 - 161/0,4 |
| 40 | 2-Norbornyl | H | H | $-CH_3$ | n-$C_6H_{13}$ | 194 - 196/0,4 |
| 41 | tert.-$C_4H_9$ | H | H | $-CH_3$ | $-CH(CH_3)C_5H_{11}$-n | $n_D^{22}$: 1,4865 |
| 42 | tert.-$C_4H_9$ | H | H | $-CH_3$ | $-CH(CH_3)CH_2CH_2CH(CH_3)_2$ | $n_D^{22}$: 1,4860 |
| 43 | tert.-$C_4H_9$ | H | H | $-CH_3$ | $-CH(C_2H_5)C_4H_9$-n | $n_D^{25}$: 1,4876 |
| 44 | tert.-$C_4H_9$ | H | H | $-CH_3$ | n-$C_8H_{17}$ | 166 - 168/0,3 |
| 45 | tert.-$C_4H_9$ | H | H | $-CH_3$ | $-CH_2-CH(CH_3)C_4H_9$-n | $n_D^{22}$: 1,4872 |
| 46 | tert.-$C_4H_9$ | H | H | $-CH_3$ | $-C(CH_3)_2CH_2C(CH_3)_3$ | $n_D^{23}$: 1,4952 |
| 47 | tert.-$C_4H_9$ | H | H | $-CH_3$ | $-CCH_2CH_2-CH(CH_3)CH_2C(CH_3)_3$ | $n_D^{22}$: 1,4871 |
| 48 | Cl | 2-Cl | H | $-CH_3$ | n-$C_{12}H_{25}$ | 190 - 194/0,1 |
| 49 | tert.-$C_4H_9$ | H | H | $-CH_3$ | $-CH_2CH=CH_2$ | |
| 50 | tert.-$C_4H_9$ | H | H | $-CH_3$ | $-CH(CH_3)CH(CH_3)_2$ | |
| 51 | tert.-$C_4H_9$ | H | H | $-CH_3$ | $-CH_2-CH_2-CH=CH_2$ | |
| 52 | tert.-$C_4H_9$ | H | H | $-CH_3$ | $-CH_2-CH=C(CH_3)_2$ | |
| 53 | tert.-$C_4H_9$ | H | H | $-CH_3$ | $-CH_2-CH_2-C(CH_3)=CH_2$ | |
| 54 | tert.-$C_4H_9$ | H | H | $-CH_3$ | -Cyclopentyl | 138 - 145/3 |
| 55 | tert.-$C_4H_9$ | H | H | $-CH_3$ | $-CH(CH_3)$Cyclopropyl | |
| 56 | tert.-$C_4H_9$ | H | H | $-CH_3$ | 2,4,4-Trimethylcyclopentyl | 145 - 153/3 |
| 57 | tert.-$C_4H_9$ | H | H | $-CH_3$ | -Cyclohexyl | 148 - 160/5 |

| Verbindung Nr. | $R^1$ | $R^2$ | $R^3$ | $R^4$ | $R^5$ | Brechungsindex oder Sdp. [$^oC$/mbar] |
|---|---|---|---|---|---|---|
| 58 | Cl | 2-Cl | H | $-CH_3$ | -Cyclohexyl | 154 - 156/0,4 |
| 59 | tert.-$C_4H_9$ | H | H | $-CH_3$ | 3-Methylcyclohexyl | 144 - 152/5 |
| 60 | tert.-$C_4H_9$ | H | H | $-CH_3$ | 4-Methylcyclohexyl | 170 - 190/5 |
| 61 | tert.-$C_4H_9$ | H | H | $-CH_3$ | 2,4-Dimethylcyclohexyl | 150 - 160/1 |
| 62 | tert.-$C_4H_9$ | H | H | $-CH_3$ | 2,5-Dimethylcyclohexyl | 150 - 160/5 |
| 63 | tert.-$C_4H_9$ | H | H | $-CH_3$ | 2,6-Dimethylcyclohexyl | 153 - 160/1 |
| 64 | tert.-$C_4H_9$ | H | H | $-CH_3$ | 3,5-Dimethylcyclohexyl | 162 - 172/5 |
| 65 | tert.-$C_4H_9$ | H | H | $-CH_3$ | 3,3,5-Trimethylcyclohexyl | 143 - 155/0,3 |
| 67 | tert.-$C_4H_9$ | H | H | $-CH_3$ | 2-Methyl-6-ethylcyclohexyl | 143 - 150/0,5 |
| 68 | tert.-$C_4H_9$ | H | H | $-CH_3$ | 2,6-Diethylcyclohexyl | 186 - 192/4 |
| 69 | tert.-$C_4H_9$ | H | H | $-CH_3$ | 4-Isopropylcyclohexyl | 200 - 210/5 |
| 70 | tert.-$C_4H_9$ | H | H | $-CH_3$ | $-CH_2$-Cyclohexyl | $n_D^{25}$: 1,5086 |
| 71 | tert.-$C_4H_9$ | H | H | $-CH_3$ | $-CH(CH_3)CH_2$-Cyclohexyl | 165 - 170/3 |
| 72 | tert.-$C_4H_9$ | H | H | $-CH_3$ | $-CH_2CH(C_2H_5)CH_2CH(CH_3)$-Cyclohexyl | 205 - 215/3 |
| 73 | tert.-$C_4H_9$ | H | H | $-CH_3$ | Cyclooctyl | $n_D^{22}$: 1,5154 |
| 74 | tert.-$C_4H_9$ | H | H | $-CH_3$ | 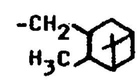 | 260 - 275/5 |
| 75 | tert.-$C_4H_9$ | H | H | $-CH_3$ | 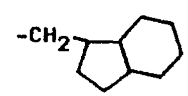 | 250 - 277/5 |

Forts. Tab. 1

| Verbindung Nr. | $R^1$ | $R^2$ | $R^3$ | $R^4$ | $R^5$ | Brechungsindex oder Sdp. [$^{o}C$/mbar] |
|---|---|---|---|---|---|---|
| 76 | tert.-$C_4H_9$ | H | H | $-CH_3$ | $-CH_2-$ 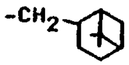 | $n_D^{22}$: 1,5127 |
| 77 | tert.-$C_4H_9$ | H | H | $-CH_3$ | $-CH_2-$ 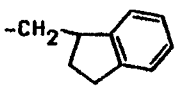 | 247 - 260/5 |
| 78 | tert.-$C_4H_9$ | H | H | $-CH_3$ | $-C_6H_5$ | 188 - 195/4 |
| 79 | tert.-$C_4H_9$ | H | H | $-CH_3$ | $4-Cl-C_6H_4-$ | 168 - 180/0,1 |
| 80 | tert.-$C_4H_9$ | H | H | $-CH_3$ | $3,4-Cl_2C_6H_3-$ | 225 - 230/4 |
| 81 | tert.-$C_4H_9$ | H | H | $-CH_3$ | $3,5-Cl_2C_6H_3-$ | 183 - 195/0,1 |
| 82 | tert.-$C_4H_9$ | H | H | $-CH_3$ | $3-(CH_3)C_6H_4-$ | 170 - 150/3 |
| 83 | tert.-$C_4H_9$ | H | H | $-CH_3$ | $4-(CH_3)C_6H_4-$ | 162 - 170/0,1 |
| 84 | tert.-$C_4H_9$ | H | H | $-CH_3$ | $2,4-(CH_3)_2C_6H_3-$ | 165 - 175/3 |
| 85 | tert.-$C_4H_9$ | H | H | $-CH_3$ | $2,5-(CH_3)_2C_6H_3-$ | 200 - 210/4 |
| 86 | tert.-$C_4H_9$ | H | H | $-CH_3$ | $2,6-(CH_3)_2C_6H_3-$ | 192 - 210/3 |
| 87 | tert.-$C_4H_9$ | H | H | $-CH_3$ | $3,5-(CH_3)_2C_6H_3-$ | 171 - 181/0,1 |
| 88 | tert.-$C_4H_9$ | H | H | $-CH_3$ | $4-(i-C_3H_7)C_6H_4-$ | 205 - 215/4 |
| 89 | tert.-$C_4H_9$ | H | H | $-CH_3$ | $2-(CH_3)$, $6-(C_2H_5)C_6H_3-$ | 215 - 218/1 |
| 90 | tert.-$C_4H_9$ | H | H | $-CH_3$ | $2,6-(C_2H_5)C_6H_3-$ | 190 - 195/3 |
| 91 | tert.-$C_4H_9$ | H | H | $-CH_3$ | $3-(CF_3)C_6H_4-$ | 160 - 170/0,3 |
| 92 | tert.-$C_4H_9$ | H | H | $-CH_3$ | $3,5-(CF_3)_2C_6H_3-$ | 170 - 180/3 |

Forts. Tab. 1

| Verbindung Nr. | $R^1$ | $R^2$ | $R^3$ | $R^4$ | $R^5$ | Brechungsindex oder Sdp. [°C/mbar] |
|---|---|---|---|---|---|---|
| 93 | tert.-$C_4H_9$ | H | H | $-CH_3$ | 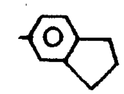 | 200 - 215/5 |
| 94 | tert.-$C_4H_9$ | H | H | $-CH_3$ | $-CH_2-C_6H_5$ | 215 - 220/5 |
| 95 | tert.-$C_4H_9$ | H | H | $-CH_3$ | $-CH(CH_3)C_6H_5$ | 160 - 170/3 |
| 96 | tert.-$C_4H_9$ | H | H | $-CH_3$ | $-CH(C_3H_7)nC_6H_5$ | 160 - 170/3 |
| 97 | tert.-$C_4H_9$ | H | H | $-CH_3$ | $4-ClC_6H_4-CH_2-$ | |
| 98 | tert.-$C_4H_9$ | H | H | $-CH_3$ | $4-(CH_3C_6H_4-CH_2-$ | |
| 99 | tert.-$C_4H_9$ | H | H | $-CH_3$ | $2,4-Cl_2C_6H_3-CH_2-$ | |
| 100 | tert.-$C_4H_9$ | H | H | $-CH_3$ | $3,4-Cl_2C_6H_3-CH_2-$ | |
| 101 | tert.-$C_4H_9$ | H | H | $-CH_3$ | $2,4-(CH_3)_2C_6H_3-$ | |
| 102 | tert.-$C_4H_9$ | H | H | $-CH_3$ | $C_6H_5-CH_2CH_2-$ | |
| 103 | tert.-$C_4H_9$ | H | H | $-CH_3$ | $C_6H_5-CH(CH_3)CH_2-$ | |
| 104 | tert.-$C_4H_9$ | H | H | $-CH_3$ | $-CH_2-$ 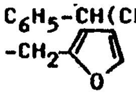 | 180 - 200/3 |
| 105 | tert.-$C_4H_9$ | H | H | $-CH_3$ | $-CH(CH_3)CH_2-C_6H_5$ | 210 - 215/3 |
| 106 | tert.-$C_4H_9$ | H | H | $-CH_3$ | $-CH(CH_3)CH(CH_3)C_6H_5$ | 172 - 180/3 |
| 107 | tert.-$C_4H_9$ | H | H | $-CH_3$ | $-CH(CH_3)CH_2CH_2C_6H_5$ | 210 - 220/1 |
| 108 | tert.-$C_4H_9$ | H | H | $-CH_3$ | $-CH_2CH_2CH(CH_3)CH_2CH_2CH_2C(CH_3)_2$ | $n_D^{22}$: 1.4966 |

Forts. Tab. 1

| Verbindung Nr. | $R^1$ | $R^2$ | $R^3$ | $R^4$ | $R^5$ | Brechungsindex oder Sdp. [°C/mbar] |
|---|---|---|---|---|---|---|
| 109 | tert.-$C_4H_9$ | H | H | $-CH_3$ | $-CH(CH_3)CH_2CH(CH_3)C_2H_5$ | $n_D^{22}$: 1,4822 |
| 110 | tert.-$C_4H_9$ | H | H | $-CH_3$ | $-CH_2-C(CH_3)_3$ | $n_D^{22}$: 1,4972 |
| 111 | tert.-$C_4H_9$ | H | H | $-CH_3$ | $-CH(C_2H_5)_2$ | 110 - 125/0,2 |
| 112 | tert.-$C_4H_9$ | H | H | $-CH_3$ | $-CH(CH_3)CH_2CH(CH_3)_2$ | 145 - 148/0,4 |

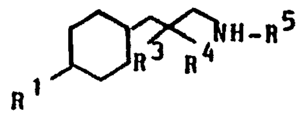

Tabelle 2

| Verbindung Nr. | $R^1$ | $R^3$ | $R^4$ | $R^5$ | Brechungsindex oder Sdp. [$^oC$/mbar] |
|---|---|---|---|---|---|
| 113 | tert.-$C_4H_9$ | H | $-CH_3$ | $-CH_3$ | |
| 114 | tert.-$C_4H_9$ | H | $-CH_3$ | $-C_2H_5$ | |
| 115 | tert.-$C_4H_9$ | H | $-CH_3$ | i-$C_3H_7$ | |
| 117 | tert.-$C_4H_9$ | H | $-CH_3$ | n-$C_5H_{11}$ | |
| 118 | tert.-$C_4H_9$ | H | $-CH_3$ | $-CH_2CH_2CH(CH_3)_2$ | |
| 119 | tert.-$C_4H_9$ | H | $-CH_3$ | $-CH(CH_3)C_4H_9$-n | |
| 120 | tert.-$C_4H_9$ | H | $-CH_3$ | n-$C_6H_{13}$ | |
| 121 | tert.-$C_4H_9$ | H | $-CH_3$ | $-CH_2CH(C_2H_5)_2$ | |
| 122 | tert.-$C_4H_9$ | H | $-CH_3$ | $-C(CH_3)_2CH(CH_3)_2$ | |
| 123 | tert.-$C_4H_9$ | H | $-CH_3$ | $-CH(CH_3)CH(CH_3)C_2H_5$ | |
| 124 | tert.-$C_4H_9$ | H | $-CH_3$ | $-CH_2CH(CH_3)C_3H_7$-n | |
| 125 | tert.-$C_4H_9$ | H | $-CH_3$ | $-CH(CH_3)C_5H_{11}$-n | |
| 126 | tert.-$C_4H_9$ | H | $-CH_3$ | $-CH(CH_3)CH_2CH_2CH(CH_3)_2$ | |
| 127 | tert.-$C_4H_9$ | H | $-CH_3$ | $-CH(C_2H_5)C_4H_9$-n | |
| 128 | tert.-$C_4H_9$ | H | $-CH_3$ | n-$C_8H_{17}$ | |
| 129 | tert.-$C_4H_9$ | H | $-CH_3$ | $-CH_2-CH(CH_3)C_4H_9$-n | |
| 130 | tert.-$C_4H_9$ | H | $-CH_3$ | $-C(CH_3)_2CH_2C(CH_3)_3$ | |
| 131 | tert.-$C_4H_9$ | H | $-CH_3$ | $C-CH_2CH_2-CH(CH_3)CH_2C(CH_3)_3$ | |

Forts. Tab. 2

| Verbindung Nr. | $R^1$ | $R^3$ | $R^4$ | $R^5$ | Brechungsindex oder Sdp. [°C/mbar] |
|---|---|---|---|---|---|
| 132 | tert.-$C_4H_9$ | H | $-CH_3$ | $-CH_2-CH=CH_2$ | |
| 133 | tert.-$C_4H_9$ | H | $-CH_3$ | $-CH_2-CH=CH-CH_3$ | |
| 134 | tert.-$C_4H_9$ | H | $-CH_3$ | $-CH_2-CH_2-CH=CH_2$ | |
| 135 | tert.-$C_4H_9$ | H | $-CH_3$ | $-CH_2-CH=C(CH_3)_2$ | |
| 136 | tert.-$C_4H_9$ | H | $-CH_3$ | $-CH_2-CH_2-C(CH_3)=CH_2$ | |
| 137 | tert.-$C_4H_9$ | H | $-CH_3$ | -Cyclopentyl | |
| 138 | tert.-$C_4H_9$ | H | $-CH_3$ | $-CH(CH_3)$Cyclopropyl | |
| 139 | tert.-$C_4H_9$ | H | $-CH_3$ | 2,4,4-Trimethylcyclopentyl | |
| 140 | tert.-$C_4H_9$ | H | $-CH_3$ | -Cyclohexyl | 160 - 163/0,3 |
| 141 | tert.-$C_4H_9$ | H | $-CH_3$ | 3-Methylcyclohexyl | |
| 142 | tert.-$C_4H_9$ | H | $-CH_3$ | 4-Methylcyclohexyl | |
| 143 | tert.-$C_4H_9$ | H | $-CH_3$ | 2,4-Dimethylcyclohexyl | |
| 144 | tert.-$C_4H_9$ | H | $-CH_3$ | 2,5-Dimethylcyclohexyl | |
| 145 | tert.-$C_4H_9$ | H | $-CH_3$ | 2,6-Dimethylcyclohexyl | |
| 146 | tert.-$C_4H_9$ | H | $-CH_3$ | 3,5-Dimethylcyclohexyl | |
| 147 | tert.-$C_4H_9$ | H | $-CH_3$ | 3,3,5-Trimethylcyclohexyl | 145 - 147/1 |
| 148 | tert.-$C_4H_9$ | H | $-CH_3$ | 2,3,6-Trimethylcyclohexyl | |
| 149 | tert.-$C_4H_9$ | H | $-CH_3$ | 2-Methyl-6-ethylcyclohexyl | |
| 150 | tert.-$C_4H_9$ | H | $-CH_3$ | 2,6-Diethylcyclohexyl | |

Forts. Tab. 2

| Verbindung Nr. | $R^1$ | $R^3$ | $R^4$ | $R^5$ | Brechungsindex oder Sdp. [°C/mbar] |
|---|---|---|---|---|---|
| 151 | tert.-$C_4H_9$ | H | $-CH_3$ | 4-Isopropylcyclohexyl | |
| 152 | tert.-$C_4H_9$ | H | $-CH_3$ | $-CH_2$-Cyclohexyl | |
| 153 | tert.-$C_4H_9$ | H | $-CH_3$ | $-CH(CH_3)CH_2$-Cyclohexyl | |
| 154 | tert.-$C_4H_9$ | H | $-CH_3$ | $-CH_2CH(C_2H_5)CH_2CH(CH_3)$-Cyclohexyl | |
| 155 | tert.-$C_4H_9$ | H | $-CH_3$ | -Cyclooctyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt-und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse -

wie Gurken, Bohnen und Kürbisgewächse -.

Die neuen Verbindungen sind insbesondere geeignet zur Bekämpfung folgender Pflanzenkrankheiten:
Erysiphe graminis an Getreide,
Erysiphe cichoracearum bzw. Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschieden Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 3 mit 10 Gewichtsteilen N-Methyl-alphapyrrolidon und erhält eine Lösung, die zur Anwendung in Form kleinster Tropfen geeignet ist.

II. 20 Gewichtsteile der Verbindung Nr. 23 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 24 werden in einer Mischung gelöst, die aus 40 Gewichtsteilen Cyolohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion, die 0,02 Gew.% des Wirkstoffs enthält.

IV. 20 Gewichtsteile der Verbindung Nr. 32 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 34 werden mit 3 Gewichtsteilen des Natriumsalzes der

Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 36 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 37 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 39 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 40 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Für die folgenden Versuche wurden als Vergleich der bekannte Wirkstoff N-Tridecyl-2,6-dimethylmorpholin (A) verwendet.

Anwendungsbeispiel 1

Wirksamkeit gegen Weizenmehltau

Blätter von in Töpfen gewachsenen Weizenkeimlingen der Sorte "Frühgold" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt.

Das Ergebnis des Versuches zeigt, daß beispielsweise bei der Anwendung als 0,025 und 0,006%ige (Gew.-%) Spritzbrühe die Verbindungen Nr. 3, 20, 23, 24, 32, 34, 36, 37, 39, 40, 41, 42 und 44 eine bessere fungizide Wirkung zeigen (97%) als der bekannte Wirkstoff A (90%).

Anwendungsbeispiel 2

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß beispielsweise bei der Anwendung als 0,025%ige Spritzbrühe die Verbindungen Nr. 20, 21, 23, 25, 26, 27, 36, 44, 70 und 140 eine bessere fungizide Wirkung zeigen (97%) als der bekannte Wirkstoff A (60 %).

Anwendungsbeispiel 3

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten,

tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß beispielsweise bei der Anwendung als 0,025%ige Spritzbrühe die Verbindungen Nr. 7, 8, 15, 16, 17, 20, 23, 24, 25, 26, 27, 28, 36, 37, 39, 41, 42, 44, 45 und 73 eine bessere fungizide Wirkung zeigen (97%) als der bekannte Wirkstoff A (50 %).

Anwendungsbeispiel 4
Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 8 Tage im Gewächshaus aufgestellt. Erst dann werden die Blätter mit einer Zoosporenaufschwemmung von Plasmopara viticola (Rebenperonospora) infiziert. Danach werden die Reben zunächst für 48 Stunden in einer wasserdampfgesättigten Kammer bei 24°C und anschließend für 6 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise bei der Anwendung als 0,05%ige Wirkstoffbrühe die Verbindungen Nr. 11, 15, 22, 23, 24, 26, 28, 31, 32, 36, 41, 44, 71 und 140 eine bessere fungizide Wirkung zeigen (97%) als der bekannte Wirkstoff A (50 %).

**Patentansprüche**

**1.** N-Arylpropylsubstituierte sekundäre Amine der Formel

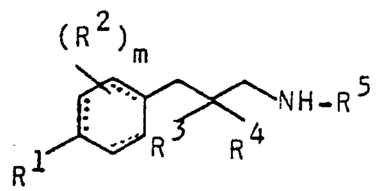

I,

worin $R^1$ und $R^2$ gleich oder verschieden sind und Wasserstoff, einen Alkylrest mit 1 - 8 Kohlenstoffatomen, einen Cycloalkylrest mit 3 bis 9 Kohlenstoffatomen, einen Halogenalkylrest mit 1 bis 3 Halogenatomen und 1 bis 4 Kohlenstoffatomen, einen Alkoxyrest mit 1 - 3 Kohlenstoffatomen, Chlor, Brom oder Fluor bedeuten, und

m die ganzen Zahlen 0, 1 oder 2 bedeuten, wobei, falls die gestrichelten Bindungen hydriert sind, $R^1$ einen $C_1$ bis $C_8$-Alkyl- oder $C_3$ bis $C_9$-Cycloalkylrest und $R^2$ Wasserstoff bedeuten, und

$R^3$ und $R^4$ gleich oder verschieden sind und Wasserstoff oder einen $C_1$ bis $C_5$-Alkylrest bedeuten, und

$R^5$ einen Alkylrest mit 1 bis 10 Kohlenstoffatomen, einen Cycloalkylrest mit 3 bis 12 Kohlenstoffatomen, einen Cycloalkenylrest mit 5 bis 12 Kohlenstoffatomen, einen Cycloalkylalkylrest mit 4 bis 13 Kohlenstoffatomen, einen Alkenylrest mit 3 bis 6 Kohlenstoffatomen, oder einen gegebenenfalls durch Halogen, $C_1$ bis $C_6$-alkyl, Halogen-$C_1$-$C_4$-alkyl, $C_1$-$C_4$-Alkoxy oder Cyan substituierten Phenyl- oder Phenyl-$C_1$-$C_6$-alkylrest oder Methylfuran bedeuten, und die gestrichelten Bindungen hydriert sein können, sowie deren Salze, außer den Verbindungen, in denen die gestrichelten Bindungen nicht hydriert sind, m die Zahl 0 und $R^5$ einen Alkyl-, Cycloalkyl-, Cycloalkylalkylrest, Bicycloheptylmethylrest oder einen gegebenenfalls durch Chlor in 4-Stellung substituierten Phenylrest bedeutet, und

außer den Verbindungen, in denen die gestrichelten Bindungen nicht hydriert sind, $R^1$ und $R^2$ Chlor, m die Zahl 1 und $R^5$ einen Alkenyl-, Cycloalkyl- oder Alkylrest bedeuten, und

außer den Verbindungen, in denen die gestrichelten Bindungen hydriert sind, m die Zahl 0 und $R^5$ einen n-Propylrest bedeutet.

**2.** Verfahren zur Herstellung von Aminen gemäß Anspruch 1, dadurch gekennzeichnet, daß man

a) einen Aldehyd der Formel II mit einem Amin der Formel III

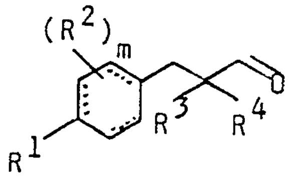 II + $H_2N-R^5$ III,

in welcher $R^1$, $R^2$, $R^3$, $R^4$, $R^5$, m und die gestrichelten Bindungen die oben genannten Bedeutungen haben, oder

b) ein Amin der Formel IV mit einem Aldehyd oder Keton der Formel V

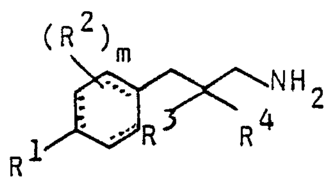 IV + O=C[R'5] V,

in welcher $R^1$, $R^2$, $R^3$, $R^4$, m und die gestrichelten Bindungen die oben genannten Bedeutungen haben und O=C[R'5] der dem Rest $R^5$ entsprechende Aldehyd oder das entsprechende Keton bedeutet, außer für die Bedeutung $R^5$ = $C_3$-$C_6$-Alkenyl, $C_5$-$C_{12}$-Cycloalkenyl, in Gegenwart von Ameisensäure oder Natriumborhydrid oder Natriumcyanborhydrid oder in in Gegenwart von Wasserstoff und einem Katalysator wie Ni, Pd oder Pt umsetzt, oder

c) eine Verbindung der Formel VI mit einem Amin der Formel III

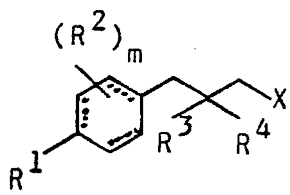 VI + III,

in welcher $R^1$, $R^2$, $R^3$, $R^4$, m, III und die gestrichelten Bindungen die oben genannten Bedeutungen haben und X für eine nucleophil verdrängbare Abgangsgruppe steht, oder

IV + X-$R^5$ VII ,

in welcher IV, X und $R^5$ die oben genannten Bedeutungen haben, umsetzt und die erhaltenen Verbindungen gegebenenfalls in ihre Salze überführt.

**3.** Fungizid, enthaltend einen festen oder flüssigen Trägerstoff und ein N-arylpropylsubstituiertes sekundäres Amin der Formel

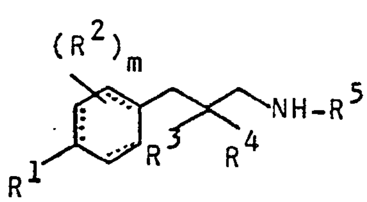 I,

worin $R^1$
und $R^2$ gleich oder verschieden sind und Wasserstoff, einen Alkylrest mit 1 - 8 Kohlenstoffatomen, einen Cycloalkylrest mit 3 bis 9 Kohlenstoffatomen, einen Halogenalkylrest mit 1 bis 3 Halogenatomen und 1 bis 4 Kohlenstoffatomen, einen Alkoxyrest mit 1 - 3 Kohlenstoffatomen, Chlor, Brom oder Fluor bedeuten, und

m die ganzen Zahlen 0, 1 oder 2 bedeuten, wobei, falls die gestrichelten Bindungen hydriert sind, $R^1$ einen $C_1$ bis $C_8$-Alkyl- oder $C_3$ bis $C_9$-Cycloalkylrest und $R^2$ Wasserstoff bedeuten, und

$R^3$ und $R^4$ gleich oder verschieden sind und Wasserstoff oder einen $C_1$ bis $C_5$-Alkylrest bedeuten, und

$R^5$ einen Alkylrest mit 1 bis 10 Kohlenstoffatomen, einen Cycloalkylrest mit 3 bis 12 Kohlenstoffatomen, einen Cycloalkenylrest mit 5 bis 12 Kohlenstoffatomen, einen Cycloalkylalkylrest mit 4 bis 13 Kohlenstoffatomen, einen Alkenylrest mit 3 bis 6 Kohlenstoffatomen, oder einen gegebenenfalls durch Halogen, $C_1$ bis $C_6$-alkyl, Halogen-$C_1$-$C_4$-alkyl, $C_1$-$C_4$-Alkoxy oder Cyan substituierten Phenyl- oder Phenyl-$C_1$-$C_6$-alkylrest oder Methylfuran bedeuten, und die gestrichelten Bindungen hydriert sein können, oder dessen Salz, außer den Verbindungen, in denen die gestrichelten Bindungen nicht hydriert sind, $R^1$ einen Isopropylrest, m die Zahl 0 und $R^5$ einen Cycloalkyl- Bicycloheptylmethylrest oder einen Alkylrest bedeutet und außer den Verbindungen, in denen die gestrichelten Bindungen hydriert sind, m die Zahl 0 und $R^5$ einen n-Propylrest bedeutet.

**4.** Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einem N-arylpropylsubstituierten sekundären Amin der Formel

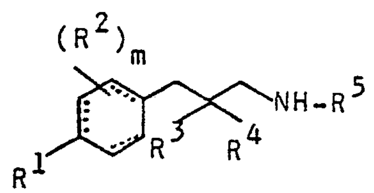 I,

worin $R^1$
und $R^2$ gleich oder verschieden sind und Wasserstoff, einen Alkylrest mit 1 - 8 Kohlenstoffatomen, einen Cycloalkylrest mit 3 bis 9 Kohlenstoffatomen, einen Halogenalkylrest mit 1 bis 3 Halogenatomen und 1 bis 4 Kohlenstoffatomen, einen Alkoxyrest mit 1 - 3 Kohlenstoffatomen, Chlor, Brom oder Fluor bedeuten, und

m die ganzen Zahlen 0, 1 oder 2 bedeuten, wobei, falls die gestrichelten Bindungen hydriert sind, $R^1$ einen $C_1$ bis $C_8$-Alkyl- oder $C_3$ bis $C_9$-Cycloalkylrest und $R^2$ Wasserstoff bedeuten, und

$R^3$ und $R^4$ gleich oder verschieden sind und Wasserstoff oder einen $C_1$ bis $C_5$-Alkylrest bedeuten, und

$R^5$ einen Alkylrest mit 1 bis 10 Kohlenstoffatomen, einen Cycloalkylrest mit 3 bis 12 Kohlenstoffatomen, einen Cycloalkenylrest mit 5 bis 12 Kohlenstoffatomen, einen Cycloalkylalkylrest mit 4 bis 13 Kohlenstoffatomen, einen Alkenylrest mit 3 bis 6 Kohlenstoffatomen, oder einen gegebenenfalls durch Halogen, $C_1$ bis $C_6$-alkyl, Halogen-$C_1$-$C_4$-alkyl, $C_1$-$C_4$-Alkoxy oder Cyan substituierten Phenyl- oder Phenyl-$C_1$-$C_6$-alkylrest oder Methylfuran bedeuten, und die gestrichelten Bindungen hydriert sein können, oder dessen Salz, außer den Verbindungen, in denen die gestrichelten Bindungen nicht hydriert sind, $R^1$ einen Isopropylrest, m die Zahl 0 und $R^5$ einen Cycloalkyl- Bicycloheptylmethylrest oder einen Alkylrest bedeutet und außer den Verbindungen, in denen die gestrichelten Bindungen hydriert sind, m die Zahl 0 und $R^5$ einen n-Propylrest bedeutet.

## Claims

**1.** An N-arylpropyl-substituted secondary amine of the formula

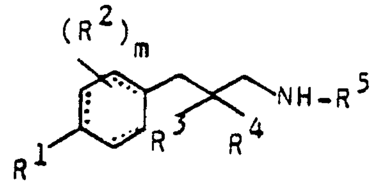

I

where $R^1$ and $R^2$ are identical or different and are each hydrogen, alkyl of 1 to 8 carbon atoms, cycloalkyl of 3 to 9 carbon atoms, haloalkyl of 1 to 3 halogen atoms and 1 to 4 carbon atoms, alkoxy of 1 to 3 carbon atoms, chlorine, bromine or fluorine, m is the integer 0, 1 or 2, $R^1$ being $C_1$-$C_8$-alkyl or $C_3$-$C_9$-cycloalkyl and $R^2$ being hydrogen where the dashed bonds are hydrogenated, and $R^3$ and $R^4$ are identical or different and are each hydrogen or $C_1$-$C_5$-alkyl and $R^5$ is alkyl of 1 to 10 carbon atoms, cycloalkyl of 3 to 12 carbon atoms, cycloalkenyl of 5 to 12 carbon atoms, cycloalkylalkyl of 4 to 13 carbon atoms, alkenyl of 3 to 6 carbon atoms or a phenyl or phenyl-$C_1$-$C_6$-alkyl radical which is unsubstituted or substituted by halogen, $C_1$-$C_6$-alkyl, halo-$C_1$-$C_4$-alkyl, $C_1$-$C_4$-alkoxy or cyano, or is methylfuran, and the dashed bonds may be hydrogenated, and salts thereof, except for the compounds in which the dashed bonds are not hydrogenated, m is 0 and $R^5$ is alkyl, cycloalkyl, cycloalkylalkyl, bicycloheptylmethyl or a phenyl radical which is unsubstituted or substituted by chlorine in the 4-position, and except for the compounds in which the dashed bonds are not hydrogenated, $R^1$ and $R^2$ are each chlorine, m is 1 and $R^5$ is alkenyl, cycloalkyl or alkyl, and except for the compounds in which the dashed bonds are hydrogenated, m is 0 and $R^5$ is n-propyl.

**2.** A process for the preparation of an amine as claimed in claim 1, wherein

a) an aldehyde of the formula II is reacted with an amine of the formula III

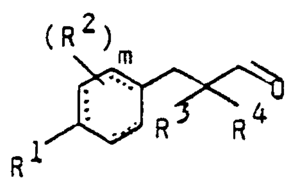

II + $H_2N$-$R^5$ III

where $R^1$, $R^2$, $R^3$, $R^4$, $R^5$, m and the dashed bonds have the abovementioned meanings, or

b) an amine of the formula IV is reacted with an aldehyde or ketone of the formula V

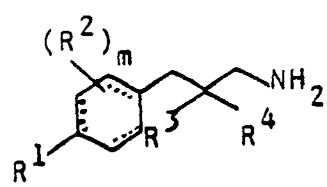

IV + O=C[R'5] V

where $R^1$, $R^2$, $R^3$, $R^4$, m and the dashed bonds have the abovementioned meanings and O=C[R'5] is the aldehyde or ketone corresponding to $R^5$, except where $R^5$ is $C_3$-$C_6$-alkenyl or $C_5$-$C_{12}$-cycloalkenyl, in the presence of formic acid or sodium borohydride or sodium cyanoborohydride or in the presence of hydrogen and a catalyst, such as Ni, Pd or Pt, or

c) a compound of the formula VI is reacted with an amine of the formula III

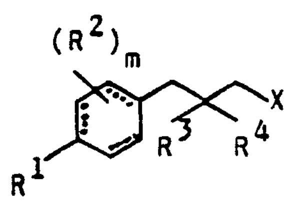

VI + III

where $R^1$, $R^2$, $R^3$, $R^4$, m, III and the dashed bonds have the abovementioned meanings and X is a leaving group which can be displaced by nucleophilic substitution, or

IV + x-$R^5$ VII

where IV, X and $R^5$ have the abovementioned meanings, and the resulting compound is, if required, converted into its salts.

**3.** A fungicide containing a solid or liquid carrier and an N-arylpropyl-substituted secondary amine of the formula

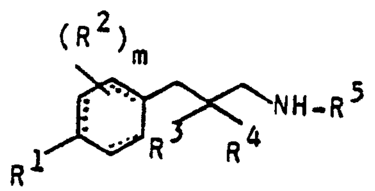

I

where $R^1$ and $R^2$ are identical or different and are each hydrogen, alkyl of 1 to 8 carbon atoms, cycloalkyl of 3 to 9 carbon atoms, haloalkyl of 1 to 3 halogen atoms and 1 to 4 carbon atoms, alkoxy of 1 to 3 carbon atoms, chlorine, bromine or fluorine, m is the integer 0, 1 or 2, $R^1$ being $C_1$-$C_8$-alkyl or $C_3$-$C_9$-cycloalkyl and $R^2$ being hydrogen where the dashed bonds are hydrogenated, and $R^3$ and $R^4$ are identical or different and are each hydrogen or $C_1$-$C_5$-alkyl and $R^5$ is alkyl of 1 to 10 carbon atoms, cycloalkyl of 3 to 12 carbon atoms, cycloalkenyl of 5 to 12 carbon atoms, cycloalkylalkyl of 4 to 13 carbon atoms, alkenyl of 3 to 6 carbon atoms or a phenyl or phenyl-$C_1$-$C_6$-alkyl radical which is unsubstituted or substituted by halogen, $C_1$-$C_6$-alkyl, halo-$C_1$-$C_4$-alkyl, $C_1$-$C_4$-alkoxy or cyano, or is methylfuran, and the dashed bonds may be hydrogenated, or a salt thereof, except for the compounds in which the dashed bonds are not hydrogenated, $R^1$ is isopropyl, m is 0 and $R^5$ is cycloalkyl, bicycloheptylmethyl or alkyl and except for the compounds in which the dashed bonds are hydrogenated, m is 0 and $R^5$ is n-propyl.

**4.** A method for controlling fungi, wherein the fungi or the materials, plants, soil or seeds to be protected from fungal attack are treated with an N-arylpropyl-substituted secondary amine of the formula

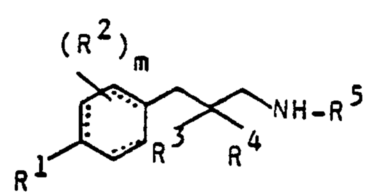

I

where $R^1$ and $R^2$ are identical or different and are each hydrogen, alkyl of 1 to 8 carbon atoms, cycloalkyl of 3 to 9 carbon atoms, haloalkyl of 1 to 3 halogen atoms and 1 to 4 carbon atoms, alkoxy of 1 to 3 carbon atoms, chlorine, bromine or fluorine, m is the integer 0, 1 or 2, $R^1$ being $C_1$-$C_8$-alkyl or $C_3$-$C_9$-cycloalkyl and $R^2$ being hydrogen where the dashed bonds are hydrogenated, and $R^3$ and $R^4$ are identical or different and are each hydrogen or $C_1$-$C_5$-alkyl and $R^5$ is alkyl of 1 to 10 carbon atoms, cycloalkyl of 3 to 12 carbon atoms, cycloalkenyl of 5 to 12 carbon atoms, cycloalkylalkyl of 4 to 13 carbon atoms, alkenyl of 3 to 6 carbon atoms or a phenyl or phenyl-$C_1$-$C_6$-alkyl radical which is unsubstituted or substituted by halogen, $C_1$-$C_6$-alkyl, halo-$C_1$-$C_4$-alkyl, $C_1$-$C_4$-alkoxy or cyano, or is methylfuran, and the dashed bonds may be hydrogenated, or a salt thereof, except for the compounds in which the dashed bonds are not hydrogenated, $R^1$ is isopropyl, m is 0 and $R^5$ is cycloalkyl, bicycloheptylmethyl or alkyl and except for the compounds in which the dashed bonds are hydrogenated, m is 0 and $R^5$ is n-propyl.

## Revendications

**1.** Amines secondaires à substituants N-arylpropyle, de formule

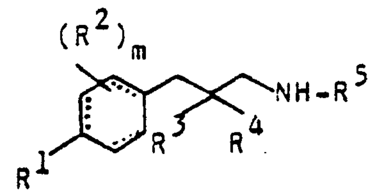

dans laquelle $R^1$ et $R^2$, ayant des significations identiques ou différentes, représentent chacun l'hydrogène, un groupe alkyle en C 1-C 8, cycloalkyle en C 3-C 9, halogénoalkyle contenant 1 à 3 atomes d'halogènes et 1 à 4 atomes de carbone, alcoxy en C 1-C 3, le chlore, le brome ou le fluor, et

m est égal à 0,1 ou 2, et, lorsque les liaisons représentées en traits interrompus sont hydrogénées, $R^1$ représente un groupe alkyle en C 1-C 8 ou cycloalkyle en C 3-C 9 et $R^2$ représente l'hydrogène, et

$R^3$ et $R^4$, ayant des significations identiques ou différentes, représentent chacun l'hydrogène ou un groupe alkyle en C 1-C 5, et

$R^5$ représente un groupe alkyle en C 1-C 10, cycloalkyle en C 3-C 12, cycloalcényle en C 5-C 12, cycloalkylalkyle en C 4-C 13, alcényle en C 3-C 6, ou un groupe phényle ou phényl-alkyle en C 1-C 6 éventuellement substitué par des halogènes, des groupes alkyle en C 1-C 6, halogènoalkyle en C 1-C 4, alcoxy en C 1-C 4 ou cyano, ou le méthylfuranne, et les liaisons représentées en traits interrompus peuvent être hydrogénées,

et leurs sels, hormis les composés pour lesquels les liaisons représentées en traits interrompus ne sont pas hydrogénées, m est égal à 0 et $R^5$ représente un groupe alkyle, cycloalkyle, cycloalkylalkyle, bicycloheptylméthyle ou un groupe phényle éventuellement substitué par le chlore en position 4, et hormis les composés pour lesquels les liaisons représentées en traits interrompus ne sont pas hydrogénées, $R^1$ et $R^2$ représentent le chlore, m est égal à 1 et $R^5$ représente un groupe alcényle, cycloalkyle ou alkyle, et hormis les composés pour lesquels les liaisons représentées en traits interrompus sont hydrogénées, m est égal à 0 et $R^5$ représente un groupe n-propyle.

**2.** Procédé de préparation des amines de la revendication 1, caractérisé en ce

a) on fait réagir un aldéhyde de formule II avec une amine de formule III

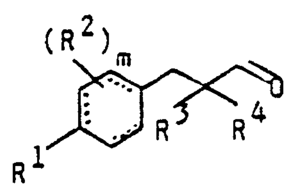 II + $H_2N-R^5$ III,

dans lesquelles $R^1$, $R^2$, $R^3$, $R^4$, $R^5$, m et les liaisons représentées en traits interrompus ont les significations indiquées ci-dessus, ou bien

b) on fait réagir une amine de formule IV avec un aldéhyde ou une cétone de formule V

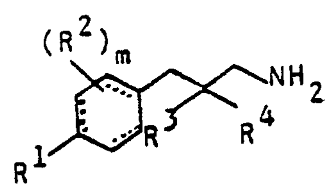 IV + O=C[R'5] V,

dans lesquelles $R^1$, $R^2$, $R^3$, $R^4$, m et les liaisons représentées en traits interrompus ont les significations indiquées ci-dessus et O=C[R'5] représente l'aldéhyde ou la cétone correspondant au radical $R^5$, sauf dans le cas ou $R^5$ représente un groupe alcényle en C 3-C 6, cycloalcényle en C 5-C 12, en présence de l'acide formique ou du borohydrure de sodium ou du cyanoborohydrure de sodium ou en présence d'hydrogène et d'un catalyseur tel que Ni, Pd ou Pt, ou bien
c) on fait réagir un composé de formule VI avec une amine de foule III

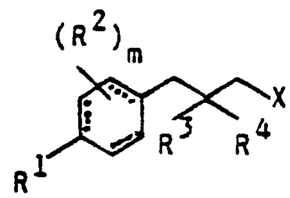 VI + III,

dans lesquelles $R^1$, $R^2$, $R^3$, $R^4$, m, III et les liaisons représentées en traits interrompus ont les significations indiquées ci-dessus et X représente un groupe éliminable nucléophile, ou bien

IV + X-$R^5$ VII,

IV, X et $R^5$ ayant les significations indiquées ci-dessus, et le cas échéant on convertit les composés obtenus en leurs sels.

3. Produit fongicide contenant un véhicule solide ou liquide et une amine secondaire à substituant N-arylpropyle de formule

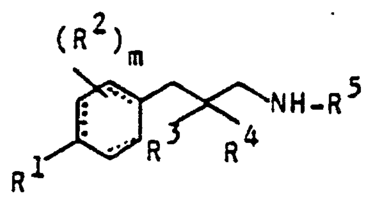 I,

dans laquelle

- $R^1$ et $R^2$, ayant des significations identiques ou différentes, représentent chacun l'hydrogène, un groupe alkyle en C 1-C 8, cycloalkyle en C 3-C9, halogénoalkyle contenant 1 à 3 atomes d'halogènes et 1 à 4 atomes de carbone, alcoxy en C 1-C 3, le chlore, le brome ou le fluor, et
- m est égal à 0,1 ou 2, et, lorsque les liaisons représentées en traits interrompus sont hydrogénées, $R^1$ représente un groupe alkyle en C 1-C 8 ou cycloalkyle en C 3-C 9 et $R^2$ représente l'hydrogène, et
- $R^3$ et $R^4$, ayant des significations identiques ou différentes, représentent chacun l'hydrogène ou un groupe alkyle en C 1-C 5, et
- $R^5$ représente un groupe alkyle en C 1-C 10, cycloalkyle en C 3-C 12, cycloalcényle en C 5-C 12, cycloalkylalkyle en C 4-C 13, alcényle en C 3-C 6, ou un groupe phényle ou phényl-alkyle en C 1-C 6 éventuellement substitué par des halogènes, des groupes alkyle en C 1-C 6 halogénoalkyle en C 1-C 4, alcoxy en C 1-C 4 ou cyano, ou le méthylfuranne, et les liaisons représentées en traits interrompus peuvent être hydrogénées,

ou un sel d'un tel composé, hormis les composés pour lesquels les liaisons représentées en traits interrompus ne sont pas hydrogénées, $R^1$ représente un groupe isopropyle, m est égal à 0 et $R^5$ représente un groupe cycloalkyl-bicycloheptylméthyle ou un groupe alkyle, et hormis les composés pour lesquels les liaisons représentées en traits interrompus sont hydrogénées, m est égal à 0 et $R^5$ représente un groupe n-propyle.

4. Procédé pour combattre les mycètes, caractérisé en ce que l'on traite les mycètes ou les matériaux, végétaux, sols ou semences à protéger contre une infection fongique, par une amine secondaire à substituant N-arylpropyle de formule

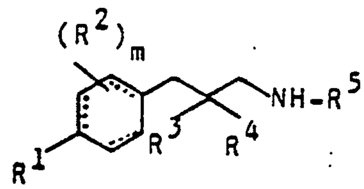

I,

dans laquelle

$R^1$ et $R^2$, ayant des significations identiques ou différentes, représentent chacun l'hydrogène, un groupe alkyle en C 1-C 8, cycloalkyle en C 3-C 9, halogénoalkyle contenant 1 à 3 atomes d'halogènes et 1 à 4 atomes de carbone, alcoxy en C 1-C 3, le chlore, le brome ou le fluor, et

m est égal à 0, 1 ou 2 et, lorsque les liaisons représentées en traits interrompus sont hydrogénées, $R^1$ représente un groupe alkyle en C 1-C 8 ou cycloalkyle en C 3-C 9 et $R^2$ représente l'hydrogène, et

$R^3$ et $R^4$, ayant des significations identiques ou différentes, représentent chacun l'hydrogène ou un groupe alkyle en C 1-C 5, et

$R^5$ représente un groupe alkyle en C 1-C 10, cycloalkyle en C 3-C 12, cycloalcényle en C 5-C 12, cycloalkylalkyle en C 4-C 13, alcényle en C 3-C 6 ou un groupe phényle ou phényl-alkyle en C 1-C 6, halogénoalkyle en C 1-C 4, alcoxy en C 1-C 4, alcoxy en C 1-C 4 ou cyano, ou le méthylfuranne, et les liaisons représentées en traits interrompus peuvent être hydrogénées,

ou un sel d'un tel composé, homis les composés pour lesquels les liaisons représentées en traits interrompus ne sont pas hydrogénées, $R^1$ représente un groupe isopropyle, m est égal à 0 et $R^5$ représente un groupe cycloalkyl-bicycloheptylméthyle ou un groupe alkyle, et hormis les composés pour lesquels les liaisons représentées en traits interrompus sont hydrogénées, m est égal à 0 et $R^5$ représente un groupe n-propyle.